# EUROPÄISCHE PATENTSCHRIFT

(11) **EP 0 378 747 B1**
(45) Veröffentlichungstag und Bekanntmachung des Hinweises auf die Patenterteilung: **20.07.1994**
(21) Anmeldenummer: 89114489.1
(22) Anmeldetag: 05.08.1989
(51) Int. Cl.: E02D 1/02

(54) **Verfahren und Entnahmegerät zur Bereitstellung von Bodenproben für Untersuchungen**
Method and soil-sampling device for preparing soil samples for examination
Procédé et dispositif de prélévement pour la préparation d'échantillon de sol à examiner

(30) Priorität: 16.01.1989 DE 3901077
(43) Veröffentlichungstag der Anmeldung: 25.07.1990
(73) Patentinhaber: FRAUNHOFER-GESELLSCHAFT ZUR FÖRDERUNG DER ANGEWANDTEN FORSCHUNG E.V., 80636 München (DE)
(72) Erfinder: Spiekermann, Martin, D-5948 Oberkirchen (DE)

(56) Entgegenhaltungen:
- EP-A- 0 173 971
- EP-A- 0 278 557
- FR-A- 2 036 451

## Beschreibung

Die Erfindung betrifft ein Verfahren und ein Entnahmegerät zur Bereitstellung von Bodenproben für Untersuchungen, bei dem Boden mit einer Stechkammer, die von einer Stützkonstruktion und einem Schneidenkranz umgeben und mit einer in Höhe des Schneidenkranzes befindlichen Trennfläche versehen ist, aus dem Erdreich entnommen wird.

Ein Verfahren und ein Entnahmegerät zur Bereitstellung von Bodenproben für Untersuchungen ist aus EP-A-0 278 557 bekannt. Ein pfahlförmiger Behälter mit einem quadratischen Querschnitt weist an seinem unteren Ende Schneiden auf. Um das untere Ende verschließen zu können, ist eine flexible Außenwand, bestehend aus einzelnen, miteinander verbundenen Stäben, an einer Seitenwand angeordnet. An der flexiblen Seitenwand sind drei Zugseile befestigt, die auf der gegenüberliegenden Wand über Rollen geführt sind. Die beiden äußeren Seile werden an Vorsprüngen vorbei und das mittlere durch einen Pfeiler eines in gleicher Höhe liegenden Versteifungskreuzes geführt.

Nachteilig ist, daß die Bereitstellung einer ungestörten Bodenprobe nicht möglich ist. Durch die Gestaltung des unteren Endes des Behälters wird das im Bereich der Schneiden abgebaute Bodenvolumen verdichtet. Das Versteifungskreuz verursacht darüber hinaus eine Teilung der Bodenprobe.

Geräte zur Bereitstellung von Bodenproben sind bekannt und genormt, vgl. DIN 19672. Die zu dem Gerät gehörende Stechkammer wird auch als Stechzylinder bezeichnet. Es wird angestrebt, mit derartigen Geräten Bodenproben so zu entnehmen, daß die Lagerung des Bodenaufbaues nicht gestört wird und sich damit eine repräsentative Probe ergibt. Derartige, aus DIN 19672 bekannte Geräte haben aber nur ein begrenztes Einsatzfeld. Sie sind nur für relativ kleine Volumina ausgelegt.

Die heutige organische und anorganische Umweltanalytik benötigt aber größere Proben, wenn sie unter anderem die Fragen der Identifizierung und Quantifizierung von Schadstoffen in der Ökosphäre bearbeitet.

Um das Verhalten und die Wege einer Substanz in der Umwelt vorhersagen zu können, müssen Kenntnisse zum Verbleib, zum Metabolismus, zum Abbau und zur Anreicherung der Substanzen im Boden vorliegen. Aussagekräftige und reproduzierbare Daten weren durch sogenannte Freiland-Lysimeterversuche erhalten, bei denen gewachsene Bodenmonolithe von ca. 0,20 bis 1,5 m³ Volumen eingesetzt werden. Das Gefüge dieser Bodenmonolithe darf durch die Entnahme selbst nicht gestört werden.

Gemäß EP-A-0 173 971 werden unter Lysimetern Gefäße aus Blech oder Kunststoff für wasser- und landwirtschaftswissenschaftliche Untersuchungen verstanden, die eine "in vivo" Untersuchung des komplexen Stoffverkehrs von boden-ökologischen Systemen ermöglichen. Lysimeter werden mit gewachsenem Boden gefüllt und in den Erdboden so eingelassen, daß sie mit der Oberfläche im Niveau des gewachsenen Erdbodens abschneiden. Derartige Lysimeter bestehen demnach aus Probenbehälter, Sickerwasserauffang- und Sammelbehälter sowie weiteren Meßvorrichtungen, die je nach Meßaufgabe zum Einsatz gelangen. Dabei soll das im Lysimeter gesammelte Sickerwasser so gesammelt werden, daß es ausschließlich nur aus der Bodenprobe stammt.

Dabei ist es wichtig, relativ große Probenflächen zu berücksichtigen. Angestrebt wird demnach eine Untersuchung, bei der eine Bodenprobe von ca. 1 m² Probenfläche bei variablen Probentiefen zwischen 0,2 m und 1,5 m in ungestörter Lage abgebaut werden kann.

Für derartige Bodenuntersuchungen gibt es nach Kenntnis des Anmelders derzeit keine Geräte.

Es stellt sich demnach die Aufgabe, für Lysimeter-Untersuchungen ein Verfahren und ein Entnahmegerät anzugeben, mit dem wesentlich voluminösere Proben entnommen und untersucht werden können, wobei die Bodenprobe derart schonend aus dem Bodenverband entnommen wird, daß sein Gefüge praktisch völlig erhalten bleibt. Gedacht ist dabei an Querschnitte von etwa 1 m² und Stichtiefen bis zu 1,5 m.

Diese Aufgabe wird mit einem Verfahren der eingangs genannten Art durch folgende Verfahrensschritte gelöst:
a) erschütterungsfreies vertikales Eindrücken der Stechkammer für die Entnahme einer voluminösen Bodenprobe von etwa 1 m² Bodenfläche in einem Boden, der sowohl bewachsen als auch frei von Bewuchs ist,
b) Abschneiden der Probe nach Erreichen einer Eindrücktiefe von 200 - 1500 mm durch ein horizontales Verschieben mit der als wenigstens eine Trennscheibe ausgebildeten Trennfläche aus dem Bodenverband,
c) Entfernen der Trennscheibe nach Trennung der Probe von der Stechkammer und Montage einer perforierten Grundplatte unter der Stechkammer und
d) Einlassen der Probe in einen Sickerauffangbehälter, der der äußeren Form der Stechkammer entspricht und eine solche Tiefe hat, daß die Probenoberfläche mit der Oberfläche des gewachsenen Erdbodens niveaugleich abschneidet.

Vorteilhaft ist es dabei, wenn ein Schürfgraben mit dem Eindrücken der Stechkammer ausgehoben wird.

Ein Entnahmegerät zur Lösung der gestellten Aufgabe ist dadurch gekennzeichnet,
- daß die Stechkammer einen Querschnitt von etwa 1 m² und eine Einstichtiefe bis zu 1500 mm hat,
- daß auf die Stützkonstruktion eine kraftaufnehmende und -verteilende Dachträgerkonstruktion mit einem zentralen Schlußstein auflegbar ist, mit der die Stechkammer erschütterungsfrei in den Boden eintreibbar ist,
- daß die Trennfläche als wenigstens eine horizontal verfahrbare Trennscheibe ausgebildet ist, mit der die Probe nach einem erschütterungsfreien Eindrücken der Stechkammer vom übrigen Boden trennbar ist, und
- daß mit einer Umsetzungsvorrichtung die Trennscheibe entfernbar und eine Grundplatte unter der Stechkammer befestigbar ist, mit der die Stechkammer mit einer in ihr befindlichen Probe in einen Sickerauffangbehälter einlaßbar ist.

Vorteilhaft ist es, wenn für die polygonartig, vorzugsweise mit zwölf gleichen Seitenflächen ausgebildete Stechkammer zwei rechteckige Trennscheiben verwendet werden.

Vorzugsweise werden hierbei die Trennscheiben an außerhalb der Stechkammer zu befestigenden Führungsschienen verschoben.

Vorteilhaft ist es, wenn der Sickerauffangbehälter so in einem gewachsenen Boden angeordnet ist, daß seine Oberfläche mit der Oberfläche der eingelassenen Bodenprobe abschneidet und wenn ein Luftspalt zwischen der Stechkammer und dem Sickerauffangbehälter so eng ist, daß in der Probe und dem gewachsenen Umgebungsboden gleiche Temperaturgradienten gewährleistet sind. Der Sickerauffangbehälter ist dabei vorteilhafterweise mit einem Sammel-Meßbehälter verbunden, wobei qualitative und quantitative Messungen durchgeführt werden können.

Merkmale weiterer Unteransprüche sind in der folgenden Beschreibung anhand der Zeichnung erläutert.

Die Figuren zeigen im einzelnen:
- Figur 1a: ein Entnahmegerät in Seitenansicht, teilweise geschnitten;
- Figur 1b: das Entnahmegerät in Draufsicht, in Schnittdarstellung entlang der Linie B ... B in Figur 1a;
- Figur 2: den untern Teil des Entnahmegerätes während des Querschneidens der Probe;
- Figur 3: zeigt die Entfernung der Trennscheiben und die Montage der Grundplatte;
- Die Fig. 4a und 4b: zeigen das Entnahmegerät in Verbindung mit einem Aufnahmebehälter in Form einer Lysimeter-Anordnung einmal im Längsschnitt (4a) und einmal im Querschnitt (4b).

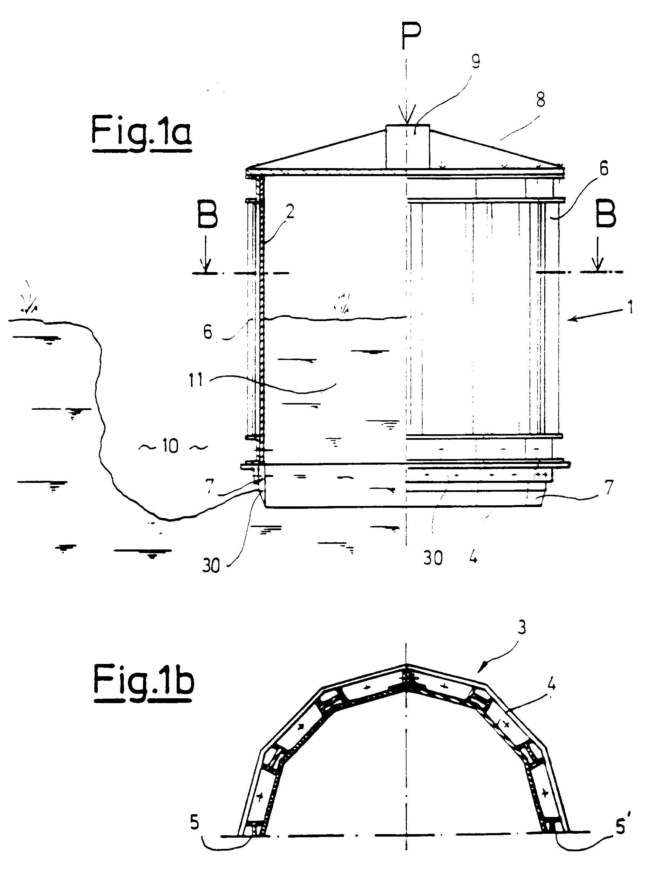

Aus den Figuren 1a und 1b geht ein Entnahmegerät 1 hervor, das zur vertikalen Entnahme von Bodenproben geeignet ist, wobei die Böden sowohl bewachsen als auch frei von Bewuchs sein können.

Das Gerät besitzt dazu eine in das Erdreich einzutreibende Stechkammer 2 als Probenbehälter.Die Stechkammer 2 ist nach oben und unten offen. Im Querschnitt handelt es sich bei der Stechkammer um einen zwölfseitigen Polygonzug, wobei dieser Polygonzug aus zwei Halbschalen 3 besteht, die jeweils sechs einzelne Wandteile umfassen. An den Stoßstellen 5, 5' sind die Halbschalen 3 seitlich miteinander verschweißt. Die Stechkammer 2 wird von außen umfangen und versteift durch eine vertikal geteilte Stützkonstruktion 6, die über entsprechende Zwischenträger die Stechkammer hält. Die Stechkammer hat eine Grundfläche von 1 m². Unterhalb des Stahlfachwerkes ist ein bündig mit der Stechkammer 2 abschließender Schneidenkranz 7 verschraubt.

Auf die Stützkonstruktion und damit auf die Stechkammer ist eine kraftaufnehmende und -verteilende Dachträgerkonstruktion 8 mit einem zentralen Schlußstein 9 aufgelegt.

Gemäß Pfeilrichtung (Pfeil P) wirkt eine Kraft, beispielsweise ein hydraulischer Stempel oder eine aufgelegte Baggerschaufel. Beim Eintreiben wird darauf geachtet, daß das Eindrücken der Stechkammer erschütterungsfrei erfolgt. Dabei können je nach Probenspezifikation Tiefen von 200 bis 1500 mm wirksam erreicht werden.

Mit dem Eindrücken der Stechkammer wird ein Schürfgraben 10 ausgehoben, um Veränderungen der Bodenschichtung zu erkennen und um das Eindringen der Stechkammer in den Boden zu erleichtern. Nach Erreichen der gewünschten Eindrücktiefe wird der Schneidenkranz 7 von der Stützkonstruktion 6 getrennt, indem die Befestigungsschrauben gelöst werden.

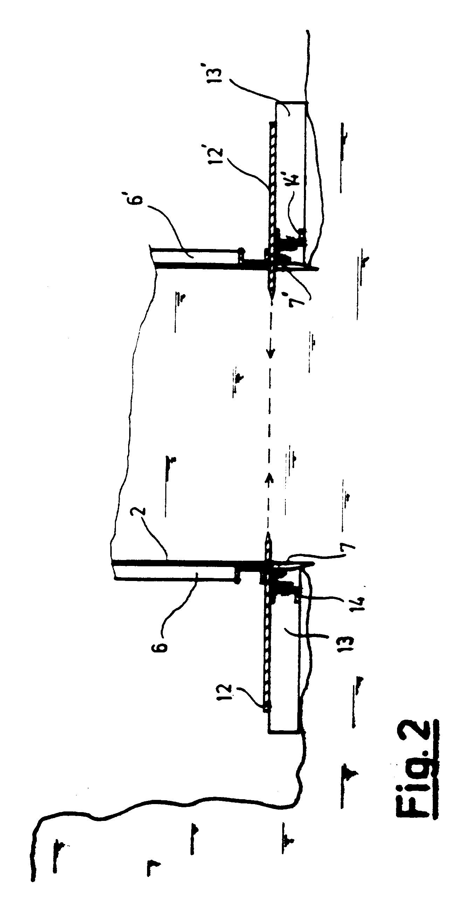

Um die sich in der Stechkammer befindliche Probe 11 aus dem Bodenverband zu trennen, sind im Bereich des Stechkranzes zwei horizontal verfahrbare Trennscheiben 12, 12' vorgesehen (vgl. Figur 2). Zur Führung dieser Trennscheiben 12, 12' werden diametral zur Stechkammer zwei Führungsschienen 13, 13' von außen an den Schneidenkranz angeschraubt. Für den späteren Abtransport der Bodenprobe 11 werden die Führungsschienen 13, 13' über zwei U-Profile 14, 14' stabilisiert. Gegen Rotation sind die Trennscheiben 12, 12' durch je zwei Leitprofile, die parallel zur schmalen Seite der Trennscheiben 12, 12', im Abstand der Führungsschienen 13, 13', an der Trennplattenunterseite angeschweißt sind, gesichert. Nach Anheben der Stützkonstruktion 6 um die Dicke der Trennscheiben 12, 12' werden diese über zwei Greifzüge, die seitlich der Trennscheiben 12, 12' montiert sind, zusammengezogen. Hierdurch wird die Probe horizontal vom Bodenverband getrennt.

Die Horizontalführung der Trennscheiben 12, 12' ist nach oben durch den unteren Rand der Stützkonstruktion 6, nach unten durch die angeschraubten Führungsschienen 13, 13' gewährleistet. Gegen vertikale Verschiebungen sind die Bauteile 6, 6' und 13, 13' im Treffbereich der Schneiden durch zwei Verschraubungen gesichert. Stützkonstruktion und Führungsschiene werden durch zwei Klauen auf maximalem Abstand der Trennscheibendicke gehalten.

Bei inhomogenen Bodengefügen im horizontalen Trennbereich kann es zu ungleichen Vorschubgeschwindigkeiten der beiden Trennscheiben 12, 12' kommen, so daß jede der Trennscheiben bei Erreichen ihrer Endposition durch zwei Stahlbolzen formschlüssig zu arretieren ist. Die Teile 13, 13' und 12, 12' sind an der Endposition der Trennscheibe fluchtend gebohrt. Wird von einer Trennscheibe die Endposition beim Schneiden des Bodens erreicht, so wird durch Einstecken eines Stahlbolzens in die beiden Bohrungen diese Trennscheibe arretiert. Zum Abtransport der Probe wird zunächst die Dachträgerkonstruktion 8 abgeschraubt und die Stützkonstruktion 6 von der Stechkammer 2 getrennt und vertikal in zwei Hälften zerlegt. Die aus dem Bodenverband getrennte Probe ist zusammen mit den Bauteilen 13, 13', 14, 14', 12, 12', 2 transportierbar.

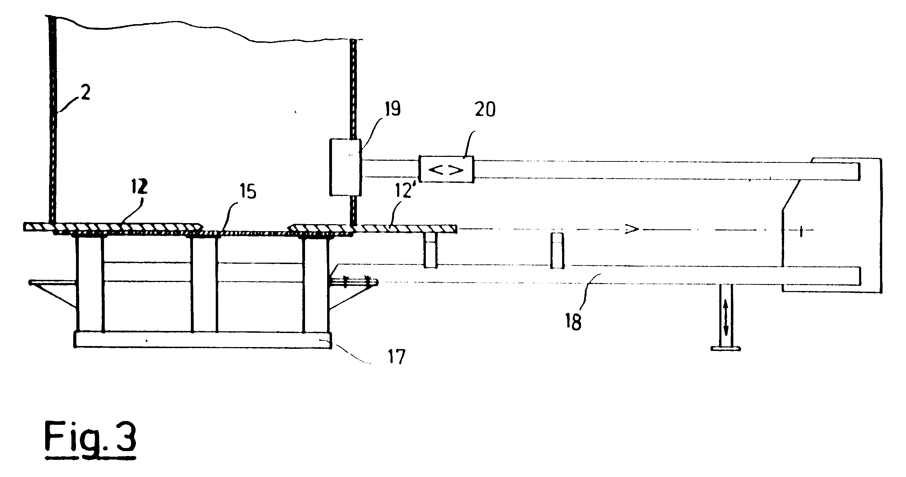

Figur 3 zeigt das Entfernen der Trennscheiben 12, 12' und die Montage einer perforierten Grundplatte 15 über Montagewinkel 16. Hierbei wurde eine Umsetzvorrichtung, bestehend aus den Teilen 17, 18, 19 und 20 konstruiert, die es ermöglicht, die perforierte Grundplatte 15 mit der die Bodenprobe enthaltende Stechkammer 2 zu verschrauben. Hierzu wird die Grundplatte 15 auf einem Montageblock 17 zentriert und arretiert. Um die Stechkammer 2 zentrisch auf die Grundplatte 15 aufsetzen zu können, wird eine Zentriereinrichtung 18 mit dem Montageblock 17 verschraubt. Nachdem die über drei Segmente mit der Stechkammer 2 formgleiche Zentrierplatte 19 entsprechend der Grundplatte 15 über eine Verschraubung 20 ausgerichtet ist, wird die aus dem Bodenverband getrennte Probe 11 zusammen mit den Bauteilen 13, 13', 12, 12', 14, 14', 2 auf die Grundplatte 15 abgesetzt. Die Führungsschienen 13, 13' und die U-Profile 14, 14' werden demontiert. Über einen Greifzug mit Festlager in der Zentriereinrichtung 18 werden die Trennscheiben 12, 12' zwischen der Grundplatte 15 und der Stechkammer 2 herausgezogen. Nachdem beide Trennscheiben 12, 12' entfernt sind, wird die Grundplatte 15 über zwölf Montagewinkel 16 mit der Stechkammer 2 verschraubt. Die Bodenprobe kann nun in den Sickerwasserauffangbehälter 21 eingelassen werden.

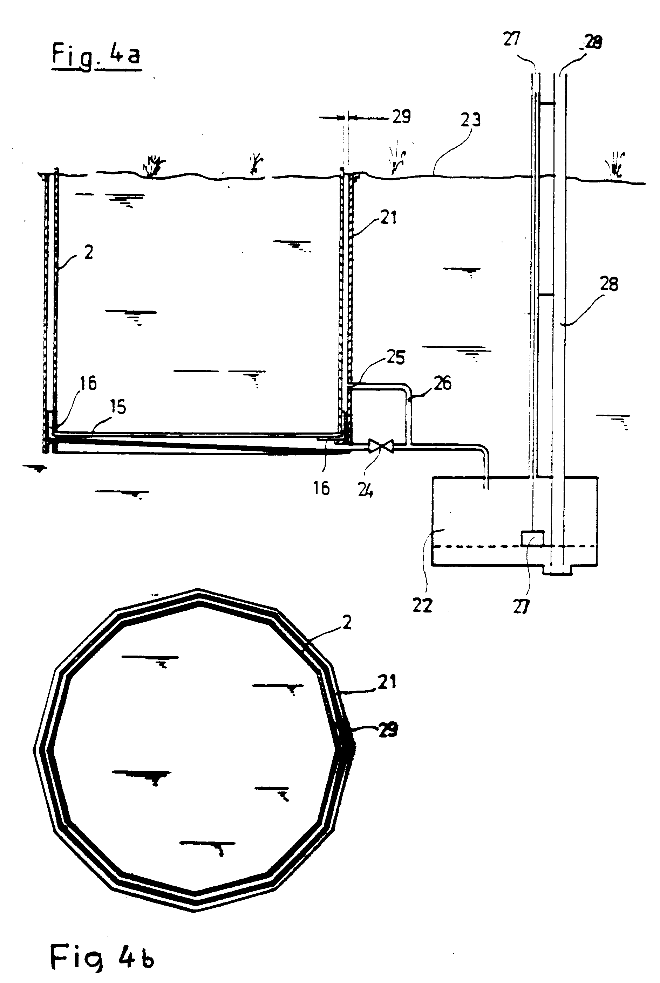

Figur 4 zeigt einen Sickerwasserauffangbehälter 21 mit einem Sickerwassersammelbehälter 22 in einem Freilandversuchsgelände. Die Auffangbehäler sind so eingegraben, daß die Probenoberfläche gemäß Figur 4 mit der Oberfläche 23 des gewachsenen Erdbodens niveaugleich abschneidet. Die Behälter 21 und 22 sind über einen flexiblen Edelstahlschlauch miteinander verbunden. Die Sickerwasserentnahmestelle wird über ein Ventil 24 geschaltet. Bei geöffnetem Ventil wird das Sickerwasser direkt abgeleitet. Bei geschlossenem Ventil entsteht ein Sumpf bis zur Anschlußhöhe 25 des zweiten Ablaufrohres 26. Der Füllstand des Sickerwassers im Sickerwassersammelbehälter 22 wird über einen Schwimmer 27 angezeigt.

Über das Absaugrohr 28 wird das Sickerwasser abgepumpt. Um gleiche Temperaturgradienten in der Bodenprobe und dem gewachsenen Umgebungsboden zu gewährleisten, sollte der Luftspalt 29 zwischen der Stechkammer 2 und dem Sickerwasserauffangbehälter 21 möglichst klein sein. Dabei wird gewährleistet, daß das gesammelte Sickerwasser ausschließlich aus der Bodenprobe stammt. Alle mit dem Sikkerwasser und der Bodenprobe in Berührung kommenden Bauteile sind aus Edelstahl gefertigt.

## Patentansprüche

1. Verfahren zur Bereitstellung von Bodenproben für Untersuchungen, bei dem Boden mit einer Stechkammer (2), die von einer Stützkonstruktion (6, 6') und einem Schneidenkranz (7) umgeben und mit einer in Höhe des Schneidenkranzes (7) befindlichen Trennfläche (12, 12') versehen ist, aus dem Erdreich entnommen wird,
gekennzeichnet durch folgende Verfahrensschritte:
a) erschütterungsfreies vertikales Eindrücken der Stechkammer (2) für die Entnahme einer voluminösen Bodenprobe (11) von etwa 1 m² Bodenfläche in einem Boden, der sowohl bewachsen als auch frei von Bewuchs ist,
b) Abschneiden der Probe (11) nach Erreichen einer Eindrücktiefe von 200 - 1500 mm durch ein horizontales Verschieben mit der als wenigstens eine Trennscheibe (12, 12') ausgebildeten Trennfläche aus dem Bodenverband,
c) Entfernen der Trennscheibe (12, 12') nach Trennung der Probe (11) von der Stechkammer (2) und Montage einer perforierten Grundplatte (15) unter der Stechkammer (2) und
d) Einlassen der Probe (11) in einen Sickerauffangbehälter (21), der der äußeren Form der Stechkammer (2) entspricht und eine solche Tiefe hat, daß die Probenoberfläche mit der Oberfläche (23) des gewachsenen Erdbodens niveaugleich abschneidet.

2. Verfahren nach Anspruch 1, dadurch gekennzeichnet, daß ein Schürfgraben (10) mit dem Eindrücken der Stechkammer (2) ausgehoben wird.

3. Verfahren nach Anspruch 1 oder 2, dadurch gekennzeichnet, daß nach dem Erreichen der gewünschten Eindringtiefe der Schneidenkranz (7) von der Stützkonstruktion (6, 6') getrennt wird.

4. Verfahren nach Anspruch 1 bis 3, dadurch gekennzeichnet, daß die Stützkonstruktion (6) um die Dicke der Trennscheiben (12, 12') angehoben wird.

5. Verfahren nach Anspruch 1 bis 4, dadurch gekennzeichnet, daß die Trennscheiben (12, 12') durch zwei diametral von außen angeschraubten Führungsschienen (13, 13') beim Abschneiden geführt und für den späteren Transport der Probe (11) stabilisiert werden.

6. Verfahren nach einem der Ansprüche 1 bis 4, dadurch gekennzeichnet, daß die Trennscheiben (12, 12') gegen Rotation durch zwei Leitprofile (14, 14') gesichert werden.

7. Verfahren nach einem der Ansprüche 1 bis 6, dadurch gekennzeichnet, daß zum Abtransport der Probe (11) wenigstens die Stützkonstruktion (6, 6') von der Stechkammer (2) getrennt wird.

8. Verfahren nach einem der Ansprüche 1 bis 7, dadurch gekennzeichnet, daß die Grundplatte (15) zum Entfernen der Trennscheiben (12, 12') auf einer Umsetzvorrichtung (17, 18, 19, 20) zentriert und arretiert wird.

9. Verfahren nach einem der Ansprüche 1 bis 8, dadurch gekennzeichnet, daß die in den Sickerauffangbehälter (21) eingelassene Probe (11) nach dem bekannten Lysimeter-Verfahren gemessen wird.

10. Entnahmegerät zur Durchführung des Verfahrens nach wenigstens einem der Ansprüche 1 bis 9, mit einer Stechkammer (2), die von einer Stützkonstruktion (6, 6') und einem Schneidenkranz (7) umgeben und mit einer in Höhe des Schneidenkranzes (7) befindlichen Trennfläche (12, 12') versehen ist, dadurch gekennzeichnet,
- daß die Stechkammer (2) einen Querschnitt von etwa 1 m² und eine Einstichtiefe bis zu 1500 mm hat,
- daß auf die Stützkonstruktion (6, 6') eine kraftaufnehmende und -verteilende Dachträgerkonstruktion (8) mit einem zentralen Schlußstein (9) auflegbar ist, mit der die Stechkammer (2) erschütterungsfrei in den Boden eintreibbar ist,
- daß die Trennfläche als wenigstens eine horizontal verfahrbare Trennscheibe (12, 12') ausgebildet ist, mit der die Probe (11) nach einem erschütterungsfreien Eindrücken der Stechkammer (2) vom übrigen Boden trennbar ist, und
- daß mit einer Umsetzungsvorrichtung (17, 18, 19) die Trennscheibe (12, 12') entfernbar und eine Grundplatte (15) unter der Stechkammer (2) befestigbar ist, mit der die Stechkammer (2) mit einer in ihr befindlichen Probe (11) in einen Sickerauffangbehälter (21) einlaßbar ist.

11. Entnahmegerät nach Anspruch 10, dadurch gekennzeichnet, daß für die polygonartig, vorzugsweise mit zwölf gleichen Seitenflächen ausgebildete Stechkammer (2) zwei rechteckige Trennscheiben (12, 12') vorgesehen sind.

12. Entnahmegerät nach Anspruch 10 oder 11, dadurch gekennzeichnet, daß die Trennscheiben (12, 12') an außerhalb der Stechkammer (2) zu befestigende Führungsschienen (13, 13') verschiebbar sind.

13. Entnahmegerät nach einem der Ansprüche 10 bis 12, dadurch gekennzeichnet, daß die eingeschobenen Trennscheiben (12, 12') im eingeschobenen Zustand feststellbar sind.

14. Entnahmegerät nach einem der Ansprüche 10 bis 13, dadurch gekennzeichnet, daß die Führungsschienen (13, 13') über zwei U-Profile (14, 14') stabilisierbar sind.

15. Entnahmegerät nach einem der Ansprüche 10 bis 14, dadurch gekennzeichnet, daß jede der Trennscheiben (12, 12') bei Erreichen ihrer Endposition durch Stahlbolzen formschlüssig arretierbar ist.

16. Entnahmegerät nach einem der Ansprüche 10 bis 15, dadurch gekennzeichnet, daß der Schneidenkranz (7) aus einzeln abnehmbaren Schneiden (13) besteht.

17. Entnahmegerät nach einem der Ansprüche 10 bis 16, dadurch gekennzeichnet, daß die Umsetzvorrichtung eine mit einem Montageblock (17) verschraubbare Zentriereinrichtung (18) und eine über drei Segmente mit der Stechkammer (2) ausrichtbare formgleiche Zentrierplatte (19) ist.

18. Entnahmegerät nach einem der Ansprüche 10 bis 17, dadurch gekennzeichnet,
- daß der Sickerauffangbehälter (21) so in einem gewachsenen Erdboden angeordnet ist, daß seine Oberfläche (23) mit der Oberfläche der eingelassenen Bodenprobe (11) abschneidet und
- daß ein Luftspalt (29) zwischen der Stechkammer (2) und dem Sickerauffangbehälter (21) so eng ist, daß in der Probe (11) und dem gewachsenen Umgebungsboden gleiche Temperaturgradienten gewährleistet sind.

19. Entnahmegerät nach Anspruch 18, dadurch gekennzeichnet, daß der Sickerauffangbehälter (21) mit einem Sammel-Meßbehälter (22) verbunden ist.

## Claims

1. A method of preparing soil samples for examination, in which the soil is taken from the ground with a punch chamber (2) which is surrounded by a support structure (6, 6') and a cutter ring (7) and is provided with a parting surface (12, 12') located at the level of the cutter ring (7), characterized by the following method steps:
a) vibrationless vertical pressing in of the punch chamber (2) for removing a voluminous soil sample (11) of approximately 1 m² ground area in ground which is both with and free from growth,
b) parting off the sample (11) from attachment to the ground, after attaining a depth of impression of 200 to 1500 mm, by horizontal displacement of the parting surface formed by at least one parting plate (12, 12'),
c) removing the parting plate (12, 12') from the punch chamber (2) after parting off the sample (11) and fitting a perforated base plate (15) under the punch chamber (2) and
d) placing the sample (11) in a leakage catch container (21) which corresponds to the outer shape of the punch chamber (2) and has such a depth that the surface of the sample intersects the level of the surface (23) of the undisturbed ground.

2. A method according to claim 1, characterized in that a trial pit (10) is dug out with the pressing in of the punch chamber (2).

3. A method according to claim 1 or 2, characterized in that, after reaching the desired depth of pressing in, the cutter ring (7) is separated from the support structure (6, 6').

4. A method according to claim 1 to 3, characterized in that the support structure (6) is raised by the thickness of the parting plates (12, 12').

5. A method according to claim 1 to 4, characterized in that the parting plates (12, 12') are guided by two guide rails (13, 13') screwed on diametrically from the outside and stabilised for subsequent transport of the sample (11).

6. A method according to any of claims 1 to 4, characterized in that the parting plates (12, 12') are secured against rotation by two guide sections (14, 14').

7. A method according to any of claims 1 to 6, characterized in that at least the support structure (6, 6') is separated from the punch chamber (2) for transporting the sample (11) away.

8. A method according to any of claims 1 to 7 , characterized in that the base plate (15) can be centred and fixed on a translating device (17, 18, 19, 20) for removal of the parting plates (12, 12').

9. A method according to any of claims 1 to 8, characterized in that the sample (11) placed in the leakage catch container (21) is measured by the known lysimeter method.

10. Sampling apparatus for carrying out the method according to at least one of claims 1 to 9, with a punch chamber (2) which is surrounded by a support structure (6, 6') and a cutter ring (7) and is provided with a parting surface (12, 12') at the level of the cutter ring (7), characterized in that
- the punch chamber (2) has a cross-section of about 1 m² and a punch-in depth of up to 1500 mm,
- a force-receiving and spreading roof support structure (8) with a central keystone (9) can be fitted to the support structure (6, 6'), by means of which the punch chamber (2) can be driven without vibration into the ground,
- the parting surface is formed from at least one horizontally movable parting plate (12, 12'), with which the sample (11) can be parted from the rest of the ground after vibrationless pressing in of the punch chamber (2), and
- the parting plate (12, 12') can be removed and a base plate (15) be fixed under the punch chamber (2) by means of a translating device (17, 18, 19), the punch chamber (2) with a sample (11) therein being capable of being placed in a leakage catch container (21) by means of the translating device.

11. Sampling apparatus according to claim 10, characterized in that two rectangular parting plates (12, 12') are provided for the polygonal punch chamber (2) preferably formed from twelve like side faces.

12. Sampling apparatus according to claim 10 or 11, characterized in that the parting plates (12, 12') are slidable on guide rails (13, 13') for fixing outside the punch chamber (2).

13. Sampling apparatus according to any of claims 10 to 12, characterized in that the inserted parting plates (12, 12') can be fixed in the inserted state.

14. Sampling apparatus according to any of claims 10 to 13, characterized in that the guide rails (13, 13') can be stabilised by two U-sections (14, 14').

15. Sampling apparatus according to any of claims 10 to 14, characterized in that each of the parting plates (12, 12') can be positively arrested on reaching its end position by steel pins.

16. Sampling apparatus according to any of claims 10 to 15, characterized in that the cutter ring (7) consists of individually removable cutters (13).

17. Sampling apparatus according to any of claims 10 to 16, characterized in that the translation device is a centring device (18) which can be screwed to a mounting block (17) and a centring plate (19) which can be aligned by means of three segments in like shape with the punch chamber (2).

18. Sampling apparatus according to any of claims 10 to 17, characterized in that,
- the leak catch container (21) is so arranged on ground with growth that its upper surface (23) coincides with the surface of the soil sample (11) placed therein and
- an air gap (29) between the punch chamber (2) and the leak catch container (21) is so narrow that like temperature gradients are ensured in the sample (11) and the surrounding ground with growth.

19. Sampling apparatus according to claim 18, characterized in that the leak catch container (21) is connected to a collecting measuring container (22).

## Revendications

1. Procédé pour l'obtention d'échantillons de sol à analyser, selon lequel on prélève des échantillons du sol au moyen d'une chambre de prélèvement (2) qui est entourée d'une construction d'appui (6, 6') et d'une couronne de coupe (7) et qui est pourvue d'une surface de séparation (12, 12') se trouvant à hauteur de la couronne de coupe (7), **caractérisé** par les étapes suivantes :
a) enfoncement vertical sans secousses de la chambre de prélèvement (2) pour le prélèvement d'un échantillon de sol volumineux (11), d'environ 1 m² de superficie, dans un sol aussi bien pourvu que dépourvu de végétation,
b) détachement de l'échantillon (11) à l'atteinte d'une profondeur de pénétration de 200 à 1500 mm, en déplaçant horizontalement dans le sol la surface de séparation réalisée sous la forme d'au moins un disque de séparation (12, 12'),
c) une fois l'échantillon (11) séparé, démontage du disque de séparation (12, 12') de la chambre de prélèvement (2), et montage d'un socle perforé (15) sous la chambre de prélèvement (2), et
d) introduction de l'échantillon (11) dans un bac collecteur d'eau d'infiltration (21), qui correspond à la forme extérieure de la chambre de prélèvement (2) et possède une profondeur telle que la surface de l'échantillon se détache de la surface (23) du sol naturel approximativement au même niveau.

2. Procédé selon la revendication 1, **caractérisé** en ce qu'une tranchée d'exploration (10) est creusée lors de l'enfoncement de la chambre de prélèvement (2).

3. Procédé selon la revendication 1 ou 2, **caractérisé** en ce qu'à l'atteinte de la profondeur de pénétration souhaitée, la couronne de coupe (7) est séparée de la construction d'appui (6, 6').

4. Procédé selon l'une quelconque des revendications 1 à 3, **caractérisé** en ce que la construction d'appui (6, 6') est relevée de l'épaisseur des disques de séparation (12, 12')

5. Procédé selon l'une quelconque des revendications 1 à 4, **caractérisé** en ce que les disques de séparation (12, 12') sont, au moyen de deux rails de guidage (13, 13') boulonnés en position diamétrale de l'extérieur, guidés lors du détachement et stabilisés en vue du transport ultérieur de l'échantillon (11).

6. Procédé selon l'une quelconque des revendications 1 à 4, **caractérisé** en ce que les disques de séparation (12, 12') sont empêchés de tourner par deux profilés directeurs.

7. Procédé selon l'une quelconque des revendications 1 à 6, **caractérisé** en ce que, pour l'évacuation de l'échantillon (11), au moins la construction d'appui (6, 6') est séparée de la chambre de prélèvement (2).

8. Procédé selon l'une quelconque des revendications 1 à 7, **caractérisé** en ce qu'en vu du démontage des disques de séparation (12, 12'), le socle (15) est centré et bloqué sur un dispositif de transfert (17, 18, 19, 20).

9. Procédé selon l'une quelconque des revendications 1 à 8, **caractérisé** en ce que l'échantillon (11) introduit dans le bac collecteur d'eau d'infiltration (21) est analysé selon le procédé connu au lysimètre.

10. Dispositif de prélèvement pour la mise en oeuvre du procédé selon l'une quelconque des revendications 1 à 9, avec une chambre de prélèvement (2) qui est entourée d'une construction d'appui (6, 6') et d'une couronne de coupe (7) et qui est pourvue d'une surface de séparation (12, 12') se trouvant à hauteur de la couronne de coupe (7), **caractérisé**
- en ce que la chambre de prélèvement (2) possède une superficie d'environ 1 m² et une profondeur de pénétration pouvant atteindre 1500 mm,
- en ce qu'on peut poser sur la construction d'appui (6, 6') un toit porteur (8) recevant et transmettant les forces, pourvu d'une clef de voûte centrale (9) permettant d'enfoncer la chambre de prélèvement (2) sans secousses dans le sol,
- en ce que la surface de séparation est réalisée sous la forme d'au moins un disque de séparation (12, 12') horizontalement déplaçable, qui permet de séparer l'échantillon (11) du reste du sol à la suite d'un enfoncement sans secousses de la chambre de prélèvement (2), et
- en ce qu'à l'aide d'un dispositif de transfert (17, 18, 19), on peut démonter le disque de séparation (12, 12') et fixer sous la chambre de prélèvement (2) un socle (15) qui permet d'introduire la chambre de prélèvement (2), contenant un échantillon (11), dans un bac collecteur d'eau d'infiltration (21).

11. Dispositif de prélèvement selon la revendication 10, **caractérisé** en ce que deux disques de séparation rectangulaires (12, 12') sont prévus pour la chambre de prélèvement (2) réalisée en forme de polygone, de préférence à douze côtés identiques.

12. Dispositif de prélèvement selon la revendication 10 ou 11, **caractérisé** en ce que les disques de séparation (12, 12') peuvent être déplacés par coulissement sur des rails de guidage (13, 13') à fixer à l'extérieur de la chambre de prélèvement (2).

13. Dispositif de prélèvement selon l'une quelconque des revendications 10 à 12, **caractérisé** en ce que les disques de séparation (12, 12') peuvent être bloqués à l'état rentré.

14. Dispositif de prélèvement selon l'une quelconque des revendications 10 à 13, **caractérisé** en ce que les rails de guidage (13, 13') peuvent être stabilisés à l'aide de deux profilés en U (14, 14').

15. Dispositif de prélèvement selon l'une quelconque des revendications 10 à 14, **caractérisé** en ce que chacun des disques de séparation (12, 12') peut, à l'atteinte de sa position finale, être bloqué en engagement positif au moyen de goujons en acier.

16. Dispositif de prélèvement selon l'une quelconque des revendications 10 à 15, **caractérisé** en ce que la couronne de coupe (7) est constituée de tranchants individuellement amovibles.

17. Dispositif de prélèvement selon l'une quelconque des revendications 10 à 16, **caractérisé** en ce que le dispositif de transfert consiste en un mécanisme de centrage (18) qui peut être boulonné sur un bloc de montage (17) et en une plaque de centrage (17), de forme identique à la chambre de prélèvement (2) et qui peut être centrée sur cette dernière par l'intermédiaire de trois segments.

18. Dispositif de prélèvement selon l'une quelconque des revendications 10 à 17, **caractérisé**
- en ce que le bac collecteur d'eau d'infiltration (21) est disposé dans un sol naturel de telle sorte que la surface (23) de ce dernier est détachée de la surface de l'échantillon de sol (11) introduit, et
- en ce que l'interstice (29) entre la chambre de prélèvement (2) et le bac collecteur d'eau d'infiltration (21) est tellement étroit que des gradients de température identiques sont garantis dans l'échantillon (11) et dans le sol naturel environnant.

19. Dispositif de prélèvement selon la revendication 18, **caractérisé** en ce que le bac collecteur d'eau d'infiltration (21) est relié à un accumulateur de mesure (22).
